(19) Europäisches Patentamt
European Patent Office
Office européen des brevets

(11) Veröffentlichungsnummer: **0 022 087**
**B1**

# (12) EUROPÄISCHE PATENTSCHRIFT

(45) Veröffentlichungstag der Patentschrift: **23.11.83**

(51) Int. Cl.[3]: **C 07 D 207/333**, C 07 D 207/34, C 08 K 5/34, C 08 L 27/04

(21) Anmeldenummer: **80810209.9**

(22) Anmeldetag: **23.06.80**

## (54) Stabilisatoren für chlorhaltige Thermoplaste.

(30) Priorität: **28.06.79 CH 6035/79**

(43) Veröffentlichungstag der Anmeldung: **07.01.81 Patentblatt 81/1**

(45) Bekanntmachung des Hinweises auf die Patenterteilung: **23.11.83 Patentblatt 83/47**

(84) Benannte Vertragsstaaten: **AT BE DE FR GB IT**

(56) Entgegenhaltungen:
**FR - A - 2 407 236**
**US - A - 3 478 053**
**US - A - 3 644 631**
**US - A - 4 111 901**

(73) Patentinhaber: **CIBA-GEIGY AG, Patentabteilung Postfach, CH-4002 Basel (CH)**

(72) Erfinder: **Wirth, Hermann O., Dr., Lessingstrasse 24, D-6140 Bensheim 3 (DE)**
Erfinder: **Büssing, Jürgen, Dr., Altengassweg 38, D-6140 Bensheim (DE)**
Erfinder: **Friedrich, Hans-Helmut, Am Rauhenstein 8, D-6147 Lautertal 2 (DE)**

EP 0 022 087 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Stabilisatoren für chlorhaltige Thermoplaste

Die vorliegende Erfindung betrifft die Stabilisierung von chlorhaltigen Thermoplasten durch Zusatz von Pyrrolen sowie neue Pyrrole.

Es ist bekannt, dass chlorhaltige Polymerisate gegen den schädigenden Einfluss von Licht und Wärme, z.B. bei der Verarbeitung zu Formteilen, geschützt werden müssen. Dazu werden bisher insbesondere Organozinnverbindungen, Metallcarboxylate oder Aminocrotonate verwendet. Die mit diesen Wirkstoffen erzielten Stabilitäten sind jedoch für die Praxis nicht immer ausreichend und es besteht ein Bedürfnis, hier Verbesserungen aufzufinden, insbesondere bessere metallfreie Thermostabilisatoren für PVC zu schaffen. Ein seit langem bekannter Stabilisator für PVC ist das 2-Phenylindol, vgl. Voigt «Stabilisierung der Kunststoffe», Springer-Verlag 1966, Seite 306, das aber nur in Kombination mit Metallcarboxylaten befriedigende Resultate liefert.

Aus den US-Patentschriften 3 404 159, 3 573 216 und 3 404 161 sind Pyrrol-malonitrile bekannt, die als UV-Absorber und Lichtschutzmittel aufgrund ihrer spezifischen Absorption zwischen 250 und 400 mµ unter anderem auch für PVC geeignet sind. Zu dem gleichen Zweck offenbart die US-Patentschrift 3 478 053 2,3-Diarylpyrrole. Zur Entwicklung verbesserter Thermostabilisatoren, die Aufgabe der vorliegenden Erfindung war, konnten diese Patentschriften nicht beitragen.

Die Erfindung betrifft chlorhaltige Thermoplaste, enthaltend ein Pyrrol der Formel I

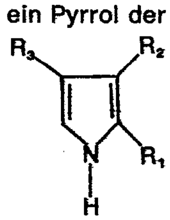

(I),

worin $R_1$ Methyl oder Phenyl ist, $R_2$ Wasserstoff, Cyano, $C_2$–$C_{19}$-Alkylcarbonyl, $C_7$–$C_{19}$-Arylcarbonyl, Phenylaminocarbonyl, m-Hydroxyphenylaminocarbonyl, α-Hydroxyäthyl oder mit einem $C_1$–$C_{18}$-Alkanol, $C_5$–$C_8$-Cycloalkanol, $C_5$–$C_{19}$-Aralkanol, $C_2$–$C_{20}$-Alkandiol, $C_4$–$C_{20}$-Thiaalkandiol oder Pentaerythrit verestertes Carboxyl ist und $R_3$ Methyl, Phenyl oder ($C_1$–$C_{18}$-Alkoxy)-methyl ist.

Es hat sich überraschenderweise gezeigt, dass die erfindungsgemäss verwendeten Pyrrole hervorragend wirksame Stabilisatoren insbesondere für PVC sind, die die Nachteile des Standes der Technik nicht oder nicht in solchem Masse aufweisen, vor allem nicht notwendig mit Metallverbindungen kombiniert werden müssen.

$R_2$ ist als $C_2$–$C_{19}$-Alkylcarbonyl insbesondere Propionyl, Butyryl, Lauroyl oder vor allem Acetyl. $R_2$ ist als $C_7$–$C_{19}$-Arylcarbonyl gegebenenfalls substituiertes Benzoyl, insbesondere Benzoyl selbst. $R_2$ ist als verestertes Carboxyl solches, das mit $C_1$–$C_{18}$-Alkanol, wie z.B. Methanol, Äthanol, n-Octanol oder Laurylalkohol, $C_5$–$C_{19}$-Aralkanol, wie z.B. Benzylalkohol oder Furfurylalkohol, $C_5$–$C_8$-Cycloalkanol, wie z.B. Cyclohexanol, $C_2$–$C_{20}$-Alkandiol, wie Äthylenglykol oder 1,2-Butylenglykol, $C_4$–$C_{20}$-Thiaalkandiol, wie z.B. 3-Thia-1,5-dihydroxy-pentan oder mit Pentaerythrit verestert ist.

$R_3$ ist als $C_1$–$C_{18}$ Alkoxymethyl, insbesondere Methoxymethyl oder Äthoxymethyl.

Bevorzugt verwendet man die in den Beispielen erwähnten Pyrrole, und von diesen insbesondere:

a) 2-Methyl-3-cyclohexyloxycarbonyl-4-phenylpyrrol,

b) 2-Methyl-3-benzyloxycarbonyl-4-phenyl-pyrrol,

c) 2-Phenyl-3-äthoxycarbonyl-4-methyl-pyrrol,

d) 2-Methyl-3-benzoyl-4-phenyl-pyrrol,

e) 2-Methyl-3-äthoxycarbonyl-4-phenyl-pyrrol,

f) 2,4-Diphenyl-pyrrol,

g) 2-Methyl-3-phenylaminocarbonyl-4-phenylpyrrol,

h) 2-Methyl-3-meta-hydroxyphenylaminocarbonyl-4-phenyl-pyrrol,

i) 2,4-Diphenyl-3-(α-hydroxy-äthyl)-pyrrol.

Pyrrole sind seit langem bekannte Verbindungen. So beschreiben Knorr und Lange in Ber. 35, 3004 (1902) die Herstellung von Pyrrolen durch Umsetzung von Aminoketonen, wie Aminoacetophenon oder Aminoaceton mit Acylaceton, wie Acetessigester oder Acetylaceton. So erhält man aus Aminoacetonen und Acetessigsäureäthylester das 2,4-Dimethyl-3-äthoxycarbonyl-pyrrol und aus Aminoacetonen und Acetylaceton das 2,4-Dimethyl-3-acetyl-pyrrol. Die Umsetzung erfolgt vorteilhaft in Gegenwart eines Puffers, wie Natriumacetat/Essigsäure. Gemäss Benary, Ber. 44, 405 (1911) kann man in obiger Reaktion das Aminoketon auch durch einen Chloraldehyd und Ammoniak ersetzen, wie durch Chloracetaldehyd und Ammoniak, z.B. als 10%ige Ammoniaklösung. Im Fall von Chloracetaldehyd erhält man 2,3-substituierte Pyrrole, z.B. aus Chloracetaldehyd, Ammoniak und Acetessigsäureäthylester das 2-Methyl-3-äthoxycarbonylpyrrol. Eine Zusammenfassung der Pyrrolchemie ist in der Monographie von R.A. Jones, «The Chemistry of Pyrroles», Academic Press 1977, publiziert.

Die Pyrrole werden den zu stabilisierenden chlorhaltigen Thermoplasten vor der Verarbeitung in üblichen Einrichtungen im allgemeinen in Mengen von 0,05 bis 5, vorzugsweise 0,1 bis 3 Gew.-% einverleibt, bezogen auf den chlorhaltigen Thermoplasten.

Zusätzlich können in üblichen Mengen herkömmliche PVC-Stabilisatoren verwendet werden, wie Organozinnstabilisatoren, Epoxyverbindungen, vorzugsweise epoxidierte Fettsäureester, wie epoxidiertes Sojabohnenöl, Phosphite, insbesondere gemischte Aryl-Alkyl-Phosphite, phenolische Antioxidantien, Metallcarboxylat-Stabilisatoren, wie Calcium-Carboxylate, insbesondere Calcium-Stearate, sowie Carboxylate von Cadmium, Zink und Barium. Costabilisatoren werden bevorzugt in Mengen von 0,05 bis 5, besonders 0,1 bis 3 Gew.-% eingearbeitet. Das Verhältnis von Pyrrol zu Costabilisatoren kann hierbei etwa 2:1 bis 1:8 betragen. Geeignete Metallstabilisatoren sind dabei die Carboxylate oder Phenolate der Metalle Barium, Calzium, Zink oder Cadmium. Die Phenole können 6 bis 20 C-Atome aufweisen und die Carbonsäuren bevorzugt 8 bis 20 C-Atome. Besonders günstig sind Mischungen von Barium- und Cadmium- oder Calcium- und Zinksalzen.

Für die erfindungsgemässen Formmassen werden bevorzugt Vinylchloridpolymere oder -copolymere verwendet. Bevorzugt sind Suspensions- und Massepolymere und ausgewaschene, also emulgatorarme Emulsionspolymere. Als Comonomere für die Copolymerisate kommen z.B. in Frage: Vinylacetat, Vinylidenchlorid, Transdichloräthan, Äthylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA und MBS.

Die Herstellung der erfindungsgemäss stabilisierten Thermoplaste erfolgt nach bekannten Verfahren durch Einarbeiten der Stabilisatoren und gegebenenfalls weiterer Stabilisatoren in das Polymerisat. Eine homogene Mischung von Stabilisator und PVC kann z.B. mit Hilfe eines Zweiwalzenmischers bei 150–210 °C erzielt werden. Je nach dem Verwendungszweck der Formmasse können vor oder mit der Einarbeitung des Stabilisators auch weitere Zusätze eingearbeitet werden, wie z.B. Gleitmittel (bevorzugt Montanwachse oder Glycerinester), Fettsäureester, Paraffine, Weichmacher, Füllstoffe, Modifikatoren (wie etwa Schlagzäh-Zusätze), Pigmente, Lichtstabilisatoren, UV-Absorber, Antioxidantien oder weitere Costabilisatoren wie z.B. Phosphite. Die erfindungsgemässen Thermoplaste können nach den dafür gebräuchlichen Formgebungsverfahren z.B. durch Extrusion, Spritzgiessen oder Kalandrieren zu Formteilen verarbeitet werden. Auch die Verwendung als Plastisole ist möglich.

Die Thermostabilisierung mit den erfindungsgemäss verwendeten Stabilisatoren ist in den erfindungsgemässen Thermoplasten hervorragend. Auch die Lichtstabilität ist gut.

Die nachfolgenden Beispiele dienen der näheren Erläuterung der Erfindung. Teile sind hierin Gewichtsteile und Prozente Gewichtsprozente.

Beispiele 1–7

Allgemeines Synthesebeispiel: Eine Mischung von 0,1 Mol α-Aminoketon-Hydrochlorid – anstelle des Aminoketonhydrochlorids kann auch ein α-Nitrosoketon eingesetzt werden, das unter den Reaktionsbedingungen – bei Verwendung eines geeigneten Reduktionsmittels – zum α-Aminoketon reduziert und simultan umgesetzt wird –, 0,12 Mol Natriumacetat und 0,1 Mol Acetessigester bzw. 1,3-Diketon werden in 100 g 75%iger Essigsäure 1 Stunde unter Rühren auf dem Wasserbad erhitzt. Nach dem Abkühlen wird das Reaktionsgemisch in kaltes Wasser eingerührt, das ausgefallene Reaktionsprodukt abgesaugt und umkristallisiert.

Bei schmierig oder flüssig anfallenden Reaktionsprodukten wird die wässrige Lösung mit Toluol bzw. Chloroform extrahiert, die toluolische Lösung über Natriumsulfat getrocknet, filtriert, mittels Rotationsverdampfer eingeengt und der Rückstand destilliert (Hochvakuum) oder umkristallisiert.

Nach diesem Verfahren wurden neben bekannten auch die folgenden noch nicht vorbeschriebenen Pyrrolderivate synthetisiert.

- 2-Methyl-3-carboxymethyl-4-phenylpyrrol (Beispiel 1, Stabilisator Nr. 10), Smp. 132–133 °C; Struktur gesichert durch 13C-NMR-Spektroskopie.
- 2-Methyl-3-carboxylauryl-4-phenylpyrrol (Beispiel 2, Stabilisator Nr. 5), Smp. 51–52 °C; Struktur gesichert durch 13C-NMR-Spektroskopie.
- 2,4-Diphenyl-3-(α-hydroxyäthyl)-pyrrol, Smp. 128 °C. (Beispiel 4).
- 2-Methyl-3-phenylaminocarbonyl-4-phenyl-pyrrol (Beispiel 5, Stabilisator Nr. 14).
- 2-Methyl-3-meta-hydroxyphenylaminocarbonyl-4-phenyl-pyrrol (Beispiel 6).
- 2-Methyl-3-cyclohexyl-oxycarbonyl-4-phenylpyrrol (Beispiel 7, Stabilisator Nr. 12).

Beispiel 8

Testdaten in Anlehnung an DIN 53 381, Bl. 3 (Dehydrochlorierungstest) von erfindungsgemässen Stabilisatoren

| | |
|---|---|
| Konz: | Konzentration bez. auf PVC (S-PVC, K-Wert 64) |
| Ind.-Zeit: | Zeit bis Anstieg der Dehydrochlorierungskurve |
| Absp.-Zeit: | Zeit bis zur Abspaltung von 0,5% des verfügbaren Chlors |
| Tangens: | Steigung der Kurve bei «Absp.-Zeit 0,5%». |

| Stabilisator Nr. | Stabilisator(en) | Konz. [%] | Ind.-Zeit [min.] | Absp.-Zeit [min] | Tangens |
|---|---|---|---|---|---|
| Vergl. | Keiner | – | 12 | 40 | 0,93 |
| 1 | 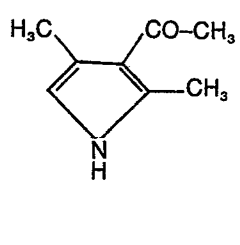 | 0,34* | 23 | 68 | 0,55 |

| Stabilisator Nr. | Stabilisator(en) | Konz. [%] | Ind.-Zeit [min.] | Absp.-Zeit [min] | Tangens |
|---|---|---|---|---|---|
| 2 | 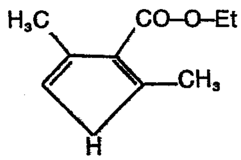 | 0,42* | 16 | 50 | 0,74 |
| 3 | 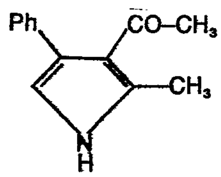 | 0,50* | 20 | 83 | 0,41 |
| 4 | 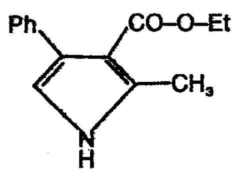 | 0,57* | 20 | 00 | 0,33 |

* entsp. 2,5 mmol pro 100 g PVC

| Stabilisator Nr. | Stabilisator(en) | Konz. [%] | Ind.-Zeit [min.] | Absp.-zeit [min.] | Tangens |
|---|---|---|---|---|---|
| Vergl. | Epoxidiertes Sojaöl = ESO | 1,0 | 19 | 52 | 0,76 |
| 4 | ESO + 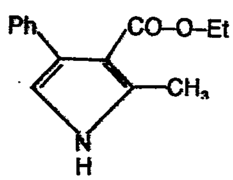 | 1,0<br>0,58 | 35 | 119 | 0,31 |
| 5 | ESO + 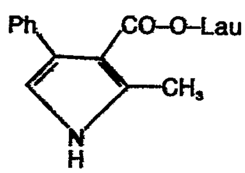 | 1,0<br>0,94 | 32 | 115 | 0,31 |
| 6 | ESO + 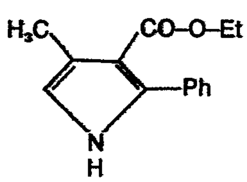 | 1,0<br>0,58 | 32 | 91 | 0,38 |
| 8 | ESO + 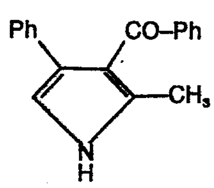 | 1,0<br>0,65 | 30 | 98 | 0,38 |

| Stabilisator Nr. | Stabilisator(en) | Konz. [%] | Ind.-Zeit [min.] | Absp.-zeit [min.] | Tangens [min] |
|---|---|---|---|---|---|
| 10 | 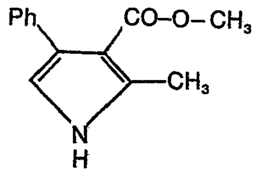 ESO + | 1,0<br>0,54 | 35 | 125 | 0,30 |
| 11 | 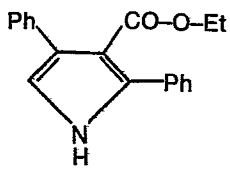 ESO + | 1,0<br>0,73 | 42 | 130 | 0,25 |
| Vergl. | Epoxidiertes Sojaöl = ESO | 2,0 | 26 | 64 | 0,67 |
| 4 | 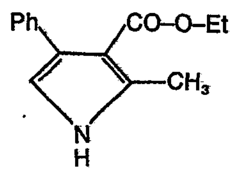 ESO + | 2,0<br>1,0 | 40 | 152 | 0,23 |
| 4 | 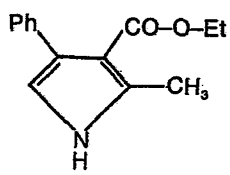 ESO +<br>Ph-O-P(-O-$C_{10}H_{21})_2$ | 2,0<br>0,75<br>0,75 | 55 | 142 | 0,30 |
| Vergl. | Epoxidiertes Sojaöl<br>Ca-Stearat<br>Zn-Stearat | 3,0<br>0,2<br>0,2 | 29 | 45 | 1,63 |
| 1 | 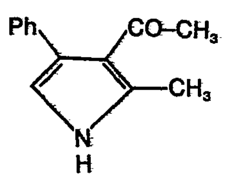 Epoxid. Sojaöl<br>Ca-Stearat<br>Zn-Stearat<br>+ | 3,0<br>0,2<br>0,2<br>0,4 | 47 | 65 | 1,44 |
| 4 | 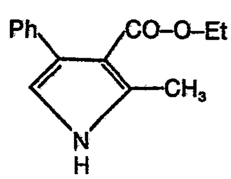 Epoxid. Sojaöl<br>Ca-Stearat<br>Zn-Stearat<br>+ | 3,0<br>0,2<br>0,2<br>0,4 | 49 | 75 | 1,0 |
| 4 | 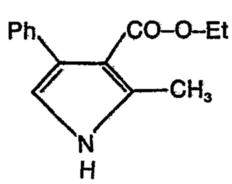 ESO<br>+<br>ESO<br>+<br>ESO<br>+ | 0,5<br>0,5<br>1,0<br>1,0<br>1,5<br>1,5 | 29<br>35<br>40 | 107<br>125<br>140 | 0,33<br>0,29<br>0,26 |

Beispiel 9

Testergebnisse von erfindungsgemässen Verbindungen im statischen Hitzetest bei 180°C.
S–PVC + 2% epoxidiertes Sojaöl + 2,5 mmol % Stabilisator.

| Stabilisator Nr. | Stabilisatoren | Yellowness-Indices Walzfell | 10 min. | 20 min. |
|---|---|---|---|---|
| 12 | 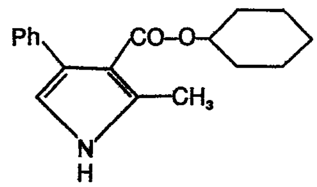 | 5,4 | 16,7 | 28,7 |
| 14 | 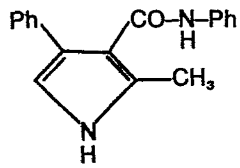 | 5,4 | 18,2 | 34 |
| 15 | 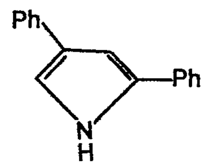 | 4,4 | 12,0 | 21,3 |

**Patentansprüche**

1. Chlorhaltige Thermoplaste, enthaltend ein Pyrrol der Formel I

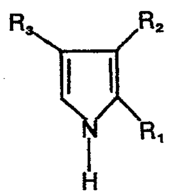

(I),

worin $R_1$ Methyl oder Phenyl ist, $R_2$ Wasserstoff, Cyano, $C_2$–$C_{19}$-Alkylcarbonyl, $C_7$–$C_{19}$-Arylcarbonyl, Phenylaminocarbonyl, m-Hydroxyphenylaminocarbonyl, α-Hydroxyäthyl oder mit einem $C_1$–$C_{18}$-Alkanol, $C_5$–$C_8$-Cycloalkanol, $C_5$–$C_{19}$-Aralkanol, $C_2$–$C_{20}$-Alkandiol, $C_4$–$C_{20}$-Thiaalkandiol oder Pentaerythrit verestertes Carboxyl ist und $R_3$ Methyl, Phenyl oder ($C_1$–$C_{18}$-Alkoxy)-methyl ist.

2. Chlorhaltige Thermoplaste nach Anspruch 1, enthaltend 2,4-Diphenyl-pyrrol.

3. Chlorhaltige Thermoplaste nach Anspruch 1, enthaltend 2-Methyl-3-cyclohexyloxycarbonyl-4-phenyl-pyrrol, 2-Methyl-3-benzyloxycarbonyl-4-phenyl-pyrrol, 2-Phenyl-3-äthoxycarbonyl-3-methyl-pyrrol, 2-Methyl-3-benzoyl-4-phenyl-pyrrol, oder 2-Methyl-3-äthoxycarbonyl-4-phenylpyrrol, 2-Methyl-3-phenylaminocarbonyl-4-phenyl-pyrrol, 2-Methyl-3-meta-hydroxyphenylaminocarbonyl-4-phenyl-pyrrol oder 2,4-Diphenyl-3-(α-hydroxyäthyl)-pyrrol.

4. Chlorhaltige Thermoplaste nach einem der Ansprüche 1 bis 3, enthaltend ein Pyrrol der Formel I in einer Menge von 0,05 bis 5 Gew.-%.

5. Chlorhaltige Thermoplaste nach einem der Ansprüche 1 bis 4, enthaltend zusätzlich einen oder mehrere herkömmliche PVC-Stabilisatoren und/oder Zusätze.

6. Chlorhaltige Thermoplaste nach einem der Ansprüche 1 bis 5, dadurch gekennzeichnet, dass das Substrat PVC ist.

7. Verwendung von Pyrrolen der Formel I gemäss Anspruch 1 zum Stabilisieren von chlorhaltigen Thermoplasten.

**Claims**

1. A chlorine-containing thermoplastic, containing a pyrrole of the formula I

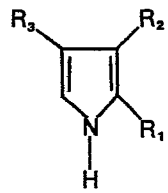

(I)

wherein $R_1$ is methyl or phenyl, $R_2$ is hydrogen, cyano, $C_2$–$C_{19}$-alkylcarbonyl, $C_7$–$C_{19}$-arylcarbonyl, phenylaminocarbonyl, m-hydroxyphenylaminocarbonyl, α-hydroxyethyl, or carboxyl which is esterified with a $C_1$–$C_{18}$-alkanol, $C_5$–$C_8$-cycloalkanol, $C_5$–$C_{19}$-aralkanol, $C_2$–$C_{20}$-alkane-diol or $C_4$–$C_{20}$-thiaalkanediol or with pentaerythritol, and $R_3$ is methyl, phenyl or ($C_1$–$C_{18}$-alkoxy)-methyl.

2. A chlorine-containing thermoplastic according to Claim 1, containing 2,4-diphenyl-pyrrole.

3. A chlorine-containing thermoplastic according to Claim 1, containing 2-methyl-3-cyclohexyloxycarbonyl-4-phenyl-pyrrole, 2-methyl-3-benzyloxycarbonyl-4-phenyl-pyrrole, 2-phenyl-3-ethoxycarbonyl-4-methyl-pyrrole, 2-methyl-3-benzoyl-4-phenyl-pyrrole, or 2-methyl-3-ethoxycarbonyl-4-phenyl-pyrrole, 2-methyl-3-phenylaminocarbonyl-4-phenyl-pyrrole, 2-methyl-3-meta-hydroxyphenylaminocarbonyl-4-phenyl-pyrrole or 2,4-diphenyl-3-(α-hydroxyethyl)-pyrrole.

4. A chlorine-containing thermoplastic according to any one of Claims 1 to 3, containing a pyrrole of the formula I in an amount of 0.05 to 5% by weight.

5. A chlorine-containing thermoplastic according to any one of Claims 1 to 4, additionally containing one or more conventional PVC stabilisers and/or additives.

6. A chlorine-containing thermoplastic according to any one of Claims 1 to 5, wherein the substrate is PVC.

7. The use of a pyrrole of the formula I according to Claim 1 for stabilising chlorine-containing thermoplastics.

**Revendications**

1. Matières thermoplastiques chlorées qui contiennent un pyrrole répondant à la formule (I):

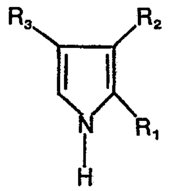

(I)

dans laquelle:

$R_1$ représente un méthyle ou un phényle,

$R_2$ représente l'hydrogène, un cyano, un alkylcarbonyle en $C_2$–$C_{19}$, un arylcarbonyle en $C_7$–$C_{19}$, un phénylaminocarbonyle, un m-hydroxy-phénylaminocarbonyle, un hydroxy-1 éthyle ou un radical carboxy estérifié par un alcanol en $C_1$–$C_{18}$, un cycloalcanol en $C_5$–$C_8$, un aralcanol en $C_5$–$C_{19}$, un alcane-diol en $C_2$–$C_{20}$, un thia-alcane-diol en $C_4$–$C_{20}$ ou le pentaérythritol, et

$R_3$ représente un méthyle, un phényle ou un alcoxyméthyle à alcoxy en $C_1$–$C_{18}$.

2. Matières thermoplastiques chlorées selon la revendication 1 qui contiennent du diphényl-2,4 pyrrole.

3. Matières thermoplastiques chlorées selon la revendication 1 qui contiennent du méthyl-2 cyclohexyloxycarbonyl-3 phényl-4 pyrrole, du méthyl-2 benzyloxycarbonyl-3 phényl-4 pyrrole, du phényl-2 éthoxycarbonyl-3 méthyl-4 pyrrole, du méthyl-2 benzoyl-3 phényl-4 pyrrole, du méthyl-2 éthoxycarbonyl-3 phényl-4 pyrrole, du méthyl-2 phénylaminocarbonyl-3 phényl-4 pyrrole, du méthyl-2 m-hydroxyphénylaminocarbonyl-3 phényl-4 pyrrole ou du diphényl-2,4 (hydroxy-1 éthyl)-3 pyrrole.

4. Matières thermoplastiques chlorées selon l'une quelconque des revendications 1 à 3, qui contiennent un pyrrole de formule (I) en une quantité de 0,05 à 5% en poids.

5. Matières thermoplastiques chlorées selon l'une quelconque des revendications 1 à 4, qui contiennent en outre un ou plusieurs stabilisants du PVC et/ou additifs usuels.

6. Matières thermoplastiques chlorées selon l'une quelconque des revendications 1 à 5, caractérisées en ce que le substrat est du PVC.

7. Application de pyrroles de formule (I) selon la revendication 1 à la stabilisation de matières thermoplastiques chlorées.